(19) Europäisches Patentamt
European Patent Office
Office européen des brevets

(11) Veröffentlichungsnummer: **0 097 615**
**B1**

(12) # EUROPÄISCHE PATENTSCHRIFT

(45) Veröffentlichungstag der Patentschrift: **21.11.85**

(51) Int. Cl.⁴: **C 07 C 121/52**, C 07 C 120/00, C 07 C 143/86, C 09 B 57/04

(21) Anmeldenummer: **83810257.2**

(22) Anmeldetag: **13.06.83**

(54) **Herstellung von Cyanobenzoesäureestern.**

(30) Priorität: **17.06.82 CH 3742/82**

(43) Veröffentlichungstag der Anmeldung: **04.01.84 Patentblatt 84/01**

(45) Bekanntmachung des Hinweises auf die Patenterteilung: **21.11.85 Patentblatt 85/47**

(84) Benannte Vertragsstaaten: **CH DE FR GB IT LI**

(56) Entgegenhaltungen:
**CH-A- 564 525**
**DE-B- 1 218 448**
**DE-C- 931 225**
**DE-C- 1 569 773**

(73) Patentinhaber: **CIBA-GEIGY AG**
**Postfach**
**CH-4002 Basel (CH)**

(72) Erfinder: **Lotz, Tobias J., Dr.**
**Unterwartweg 11**
**CH-4132 Muttenz (CH)**

**EP 0 097 615 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Ein bekanntes Verfahren (GB-PS 1 319 731) zur Herstellung von 3,4,5,6-Tetrachlor-2-cyanobenzoesäuremethylester besteht darin, ausgehend von Tetrachlorphthalsäureanhydrid das Ammoniumsalz der 3,4,5,6-Tetrachlor-2-cyanobenzoesäure herzustellen und dieses in Gegenwart eines säurebindenden Mittels zu verestern. Auf der Suche nach einem arbeitssparenderen, weniger aufwendigen und umweltfreundlicheren Verfahren wurde gefunden, dass man auf einfache Weise in hoher Ausbeute 3,4,5,6-Tetrachlor-2-cyanobenzoesäuremethylester erhält, wenn man 3,4,5,6-Tetrachlor-2-methoxycarbonylbenzoesäure als Ausgangsprodukt wählt und dieses nach an sich bekannten Verfahren (DE-PS 931 225, DE-PS 1 218 448) mit Chlorsulfonylisocyanat und Dimethylformamid zur gewünschten Verbindung umsetzt. Dass sich 3,4,5,6-Tetrachlor-2-methoxycarbonylbenzoesäuremethylester mit Chlorsulfonylisocyanat in so hohen Ausbeuten umsetzen lässt, ist überraschend. Gemäss Angew. Chem. *80*, S. 183 (1968) erschweren oder verhindern nämlich elektronenziehende Substituenten die Umsetzung einer aromatischen Säure mit Chlorsulfonylisocyanat.

Die Erfindung betrifft somit ein Verfahren zur Herstellung von Verbindungen der Formel I

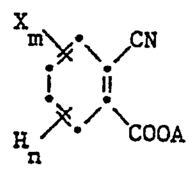

(I)

worin X Halogen, A Alkyl oder Phenyl bedeuten, m die Zahl 1 bis 4, und n die Zahl 0 bis 3 bedeuten, wobei die Summe von m + n 4 beträgt, dadurch gekennzeichnet, dass man die Verbindung der Formel II

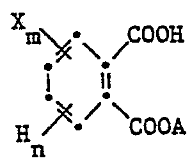

(II)

worin die Symbole X, A, m und n die oben angegebenen Bedeutungen haben, in alleiniger Gegenwart von einer mindestens stöchiometrischen Menge Chlorsulfonylisocyanat zur Verbindung der Formel III umsetzt

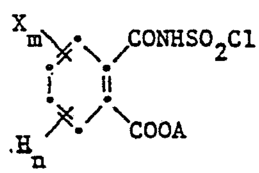

(III)

worin die Symbole X, A, m und n die oben angegebenen Bedeutungen haben, und die so erhaltene Verbindung der Formel III mittels eines Carbonsäureamides in die Verbindung der Formel I überführt.

X als Halogen kann Fluor, Chlor oder Brom, bevorzugt Chlor bedeuten.

A als Alkyl kann verzweigt oder unverzweigt sein und bevorzugt 1 bis 8, vor allem 1 bis 4 C-Atome enthalten, wie beispielsweise Methyl, Aethyl, n-Propyl, Isopropyl, n-Butyl, sec.-Butyl, tert.-Butyl, tert.-Amyl, n-Pentyl, n-Hexyl, 1,1,3,3-Tetramethylbutyl, n-Heptyl oder n-Octyl.

Bedeutet A Phenyl, so kann es sich um durch Halogen, wie Chlor, $C_1$-$C_4$-Alkyl, wie Methyl, Aethyl, Isopropyl oder tert.-Butyl, oder $C_1$-$C_4$-Alkoxy, wie Methoxy, Aethoxy, Isopropoxy oder Butoxy substituiertes Phenyl handeln, wie beispielsweise um 2-Methylphenyl, 4-Chlorphenyl, 2-Methoxyphenyl oder 2,4-Dichlorphenyl. Phenyl als Bedeutung von A ist aber bevorzugt unsubstituiert.

Vorzugsweise verwendet man zur erfindungsgemässen Herstellung von Verbindungen der Formel I als Ausgangsstoff eine Verbindung der Formel II, worin X Chlor, A $C_1$-$C_4$-Alkyl oder unsubstituiertes Phenyl, m eine Zahl von 2 bis 4 und n eine Zahl von Null bis 2 bedeuten.

Besonders bevorzugt verwendet man als Ausgangsstoffe Verbindungen der Formel II, worin X Chlor, A Methyl, m eine Zahl von 2 bis 4, und n eine Zahl von Null bis 2 bedeuten.

Von besonderem Interesse als Ausgangsstoff der Formel II ist 3,4,5,6-Tetrachlor-2-methoxycarbonylbenzoesäure.

Die Umsetzung der Verbindung der Formel II zur Verbindung der Formel III in Gegenwart von Chlorsulfonylisocyanat erfolgt auf an sich bekannte Weise, indem man die Säure der Formel II mit mindestens stöchiometrischen Mengen Chlorsulfonylisocyanat zusammenbringt. Verwendet man aber

das Chlorsulfonylisocyanat in solch einem Ueberschuss, dass man ein gut rührbares Reaktionsgemisch erhält, so wird die Ausbeute günstig beeinflusst. Somit kann man das Chlorsulfonylisocyanat bevorzugt in Mengen von 3 bis 10, vor allem 4,5 bis 5,5 Moläquivalenten, bezogen auf die umzusetzende Säure, einsetzen. Es hat sich zudem gezeigt, das sich die Zugabe eines weiteren Lösungsmittels in der Regel ungünstig auf die Ausbeute auswirkt, so dass man die Umsetzung der Verbindung der Formel II zur Verbindung der Formel III nur in Gegenwart von Chlorsulfonylisocyanat durchführt.

Die Umsetzung der Verbindung der Formel II zur Verbindung der Formel III kann bei Temperaturen zwischen 0° und 100 °C erfolgen. In der Regel bevorzugt man jedoch Temperaturen zwischen 20° und 60 °C, insbesondere 30-50 °C.

Die Verbindungen der Formel III lassen sich auf bekannte Weise isolieren. Ist die Umsetzung der Verbindung der Formel II zur Verbindung der Formel III beendet, so wird das entstandene Produkt in der Regel jedoch nicht isoliert, sondern unmittelbar zum Nitril der Formel I weiterverarbeitet. Wurde das Chlorsulfonylisocyanat im Ueberschuss eingesetzt, so empfiehlt es sich in der Regel, dieses zu entfernen, beispielsweise durch Destillation, bevor die Verbindung der Formel III nach an sich bekannten Verfahren mit einem Carbonsäureamid umgesetzt wird. Zur Destillation des Chlorsulfonylisocyanates kann man zweckmässig ein höhersiedendes Lösungs- oder Verdünnungsmittel zugeben, das nachher für die Umsetzung geeignet ist und im Reaktionsgemisch belassen werden kann.

Geeignete Carbonsäureamide sind beispielsweise Formamid, Dimethylformamid, N-Methylformanilid, Diphenylformamid, Diäthylacetamid, N-Acetylmorpholin, N-Butyrylpiperidin, Essigsäurecyclohexylamid, Benzoesäuredimethylamid, Phenylessigsäurepropylamid, Brenzschleimsäuredimethylamid, Adipinsäure-bis-dimethylamid, Sebacinsäure-bis-monobutylamid, N,N'-Dipropionyl-hexamethylendiamin, Lactame, wie 4,4-Dimethylacetidinon, α-Pyrrolidon, N-Methyl-α-pyrrolidon, Caprolactam, sowie auch Polyamide, wie sie beispielsweise durch Polykondensation von Diaminen mit Dicarbonsäuren oder Lactamen oder durch Polymerisation von Acrylamiden, N-Vinylamiden oder N-Vinyllactamen entstehen.

Bevorzugt verwendet man im erfindungsgemässen Verfahren flüssige Carbonsäureamide, wie Dimethylformamid oder N-Methylpyrrolidon. Besonders bevorzugt verwendet man Dimethylformamid.

Das Carbonsäureamid wird in einer mindesten stöchiometrischen Menge, bezogen auf die Verbindung der Formel III, eingesetzt. Verwendet man kein weiteres Lösungs- oder Verdünnungsmittel, so setzt man bevorzugt einen Ueberschuss an Carbonsäureamid ein, um ein gut rührbares Reaktionsgemisch zu erhalten. Liegt ein Lösungs- oder Verdünnungsmittel vor, so verwendet man bevorzugt 1,2 bis 2 Moläquivalente Carbonsäureamid.

Die Umsetzung der Verbindung der Formel III mit dem Carbonsäureamid kann bei einer Temperatur von −30° bis 120 °C, vorzugsweise von −5° bis 50 °C, vor allem von 0° bis 20 °C, erfolgen.

Bei der Umsetzung der Verbindung der Formel III mit dem Carbonsäureamid kann man ein bei der durchzuführenden Reaktion inertes Lösungs- oder Verdünnungsmittel zusetzen. Geeignete Lösungs- bzw. Verdünnungsmittel sind beispielsweise Benzol, Toluol, Xylole, Kresole, Methylenchlorid, Tetrachlorkohlenstoff, Tetrahydrofuran oder Tetramethylensulfon. Bevorzugt verwendet man im erfindungsgemässen Verfahren als Lösungs- bzw. Verdünnungsmittel Gemische von aliphatischen, insbesondere aromatischen Kohlenwasserstoffen.

Nach der Umsetzung mit dem Carbonsäureamid können die Verbindungen der Formel I auf bekannte Art isoliert werden. In der Regel ist es möglich, die erfindungsgemäss erhaltenen Produkte ohne weitere Reinigung zu verwenden. Je nach Verwendungszweck kann es von Vorteil sein, die Verbindungen der Formel I nach üblichen Verfahren, wie Destillieren, Kristallisieren oder Schmelzen, zu reinigen.

Die Ausgangsverbindungen der Formel II lassen sich nach bekannten Verfahren herstellen, wie z. B. durch Umsetzen eines mit 1 bis 4 Halogenatomen substituierten Phthalsäureanhydrids mit einem Alkohol (vgl. JACS *69*, 2679 (1947)).

Die Verbindungen der Formel III sind neu und stellen ebenfalls einen Gegenstand der vorliegenden Erfindung dar.

Die Verbindungen der Formel I sind bekannt und eignen sich als Ausgangsstoffe zur Herstellung von hochwertigen Pigmenten der Isoindolinonreihe für hochmolekulares organisches Material. Die Verbindungen der Formel I können beispielsweise mit einer aktiven Methylenverbindung, wie Cyanacetanilid oder Barbitursäure oder mit einem aromatischen Amin oder Diamin umgesetzt werden.

In den folgenden Beispielen bedeuten Prozente Gewichtsprozente.

## Beispiel 1

Man erwärmt eine Suspension aus 159,0 g (0,5 Mol) 3,4,5,6-Tetrachlorphthalsäuremethylester und 354 g (2,5 Mol) Chlorsulfonylisocyanat unter Rühren auf etwa 32 °C. Nach Beginn der Kohlendioxidabspaltung erhält man vorübergehend eine Lösung, dann wieder eine Suspension. Zum vollständigen Umsatz erwärmt man auf 50 °C, bis die Kohlenstoffdioxidabspaltung beendet ist. Nun fügt man 300 ml Solvesso 100® (Esso) zu und destilliert in Vakuum das überschüssige Chlorsulfonylisocyanat ab. Nach dem Abkühlen auf 0-5 °C tropft man 109,5 g (1,5 Mol) Dimethylformamid so zu, dass die Temperatur des Reaktionsgemisches 15 °C nicht übersteigt. Nach beendeter Zugabe erwärmt man die entstandene Lösung 30 Minuten bei 60 °C, kühlt auf 0-5 °C ab und tropft so 73 g Wasser zu, dass die Innentemperatur 15 °C nicht übersteigt. Nach beendetem Zulauf rührt man 30 Minuten bei 60 °C und trennt anschliessend

die wässerige Phase ab. Die das Reaktionsprodukt enthaltende Phase wird mit wässeriger Natronlauge gewaschen. Nach dem Abdestillieren des Solvesso 100® im Vakuum erhält man 127,6 g (83 % der Theorie, bezogen auf den eingesetzen 3,4,5,6-Tetrachlorphthalsäuremethylester) reinen 3,4,5,6-Tetrachlor-2-cyanobenzoesäuremethylester (Schmelzpunkt : 77-82 °C).

Beispiel 2

Man erwärmt eine Suspension aus 118,4 g (0,372 Mol) 3,4,5,6-Tetrachlorphthalsäuremethylester und 194,5 g (1,374 Mol) Chlorsulfonylisocyanat unter Rühren auf etwa 47 °C. Nach Beginn der Kohlendioxidabspaltung kühlt man auf 34°-37 °C ab und erhält im Laufe der Reaktion vorübergehend eine Lösung, dann wieder eine Suspension. Zum vollständigen Umsatz erwärmt man nochmals auf 52-55 °C, bis die $CO_2$-Abspaltung beendet ist. Nun fügt man 300 ml Cyclohexan zu. Nach dem Abkühlen auf 0°-5 °C filtriert man die weisse Suspension ab und wäscht nochmals mit 250 ml Cyclohexan gut nach. Nach dem Trocknen im Vakuum erhält man 135,2 g (87,5 % der Theorie) des Zwischenproduktes der Formel

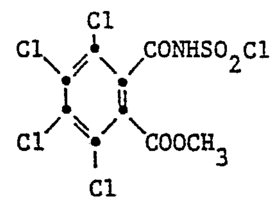

(IV)

50 g des Zwischenproduktes der Formel (IV) in 125 ml Toluol werden auf 0°-5 °C abgekühlt und tropfenweise mit 20 ml Dimethylformamid versetzt. Nach beendeter Zugabe erwärmt man die entstandene Lösung 20 Minuten auf 50°-55 °C, kühlt auf 0°-5 °C ab und tropft zu dieser Suspension 20 ml Wasser zu. Nach beendetem Zulauf wird das Reaktionsgemisch auf 60°-65 °C erwärmt, bis alles in Lösung gegangen ist. Die wässrige Phase wird abgetrennt und die Toluol-Phase wäscht man mit 40 ml 50 %-igem wässerigem Dimethylformamid, dann mit 25 ml einer 5 %-igen wässerigen Natriumbicarbonat-Lösung und schliesslich mit 40 ml einer 10 %-igen wässerigen Kochsalzlösung. Nach einer Aktivkohlenbehandlung und Klärfiltration dampft man zur Trockene ein. Man erhält 34,7 g 3,4,5,6-Tetrachlor-2-cyano-benzoesäuremethylester (Schmelzpunkt 83-85 °C).

Beispiel 3

Man erwärmt eine Suspension aus 159,0 g (0,5 Mol) 3,4,5,6-Tetrachlorphthalsäuremethylester und 424,5 g (3,0 Mol) Chlorsulfonylisocyanat unter Rühren auf etwa 30 °C. Nach Beginn der Kohlendioxidabspaltung erhält man vorübergehend eine Lösung, dann wieder eine Suspension. Zum vollständigen Umsatz erwärmt man auf 52 °C, bis die Kohlenstoffdioxidabspaltung beendet ist. Nun fügt man 300 ml Solvesso 100® (Esso) zu und destilliert in Vakuum das überschüssige Chlorsulfonylisocyanat ab. Nach dem Abkühlen auf 0-5 °C tropft man 109,5 g (1,5 Mol) Dimethylformamid so zu, dass die Temperatur des Reaktionsgemisches 15 °C nicht übersteigt. Nach beendeter Zugabe erwärmt man die entstandene Lösung 15 Minuten bei 60 °C, kühlt auf 0-5 °C ab und tropft so 73 g Wasser zu, dass die Innentemperatur 15 °C nicht übersteigt. Nach beendetem Zulauf rührt man 30 Minuten bei 70 °C und trennt anschliessend die wässerige Phase ab. Die das Reaktionsprodukt enthaltende Phase wird mit wässeriger Natronlauge bei 75 °C gewaschen. Nach dem Abdestillieren des Solvesso 100® im Vakuum erhält man 114,6 g (76,6 % der Theorie, bezogen auf den eingesetzten Tetrachlorphthalsäuremethylester) reinen 3,4,5,6-Tetrachlor-2-cyanobenzoesäuremethylester (Schmelzpunkt : 77-82 °C).

Beispiel 4

20 g 3,4,5,6-Tetrachlor-2-Cyanbenzoesäuremethylester werden mit 68,5 ml einer 1 N Natriummethylatlösung in Methanol zu einer klaren Lösung verrührt. Dabei entsteht das Natriumsalz des 3,3-Dimethoxy-4,5,6,7-tetrachlorisoindolin-1-ons. Nun werden 7 g 2,4-Diamino-3',4'-dichlorazobenzol und 100 ml o-Dichlorbenzol eingerührt. Unter Abdestillieren von Methanol und gutem Rühren wird das Reaktionsgemisch auf 100 °C geheizt. Nach Zusatz von weiteren 100 ml o-Dichlorbenzol und 24 ml Eisessig wird die Temperatur auf 140 bis 150 °C gesteigert und 2 Stunden gehalten. Der unlösliche Farbstoff wird bei 120 °C abfiltriert und mit Alkohol, Aceton und Wasser gewaschen. Nach dem Trocknen erhält man 19 g eines Gelbpigmentes, das in dieser Form direkt zur Einarbeitung in Lacke verwendet werden kann. Die Lackierungen zeichnen sich durch hervorragende Licht- und Wetterechtheiten aus.

**Patentansprüche**

1. Verfahren zur Herstellung von Verbindungen der Formel I

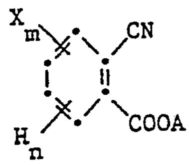

(I)

worin X Halogen, A Alkyl oder Phenyl bedeuten, m die Zahl 1 bis 4, und n die Zahl 0 bis 3 bedeuten, wobei die Summe von m + n 4 beträgt, dadurch gekennzeichnet, dass man die Verbindung der Formel II

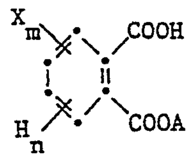

(II)

worin die Symbole X, A, m und n die oben angegebene Bedeutung haben, in alleiniger Gegenwart von einer mindestens stöchiometrischen Menge Chlorsulfonylisocyanat zur Verbindung der Formel III umsetzt

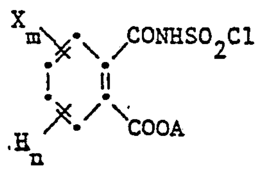

(III)

worin die Symbole X, A, m und n die oben angegebene Bedeutung haben und die so erhaltene Verbindung der Formel III mittels eines Carbonsäureamides in die Verbindung der Formel I überführt.

2. Verfahren nach Anspruch 1, dadurch gekennzeichnet, dass man als Ausgansstoff eine Verbindung der Formel II verwendet, worin X Chlor, A $C_1$-$C_4$-Alkyl oder unsubstituiertes Phenyl, m eine Zahl von 2 bis 4, und n eine Zahl von Null bis 2 bedeuten.

3. Verfahren nach Anspruch 1, dadurch gekennzeichnet, dass man als Ausgangsstoff eine Verbindung der Formel II verwendet, worin X Chlor, A Methyl, m eine Zahl von 2 bis 4, und n eine Zahl von Null bis 2 bedeuten.

4. Verfahren nach Anspruch 1, dadurch gekennzeichnet, dass man als Ausgangsstoff 3,4,5,6-Tetrachlor-2-methoxycarbonylbenzoesäure verwendet.

5. Verfahren nach Anspruch 1, dadurch gekennzeichnet, dass man das Chlorsulfonylisocyanat in Mengen von 3 bis 10 Moläquivalenten bezogen auf die umzusetzende Säure einsetzt.

6. Verfahren nach Anspruch 1, dadurch gekennzeichnet, dass man die Verbindung der Formel II bei einer Temperatur von 20 bis 60 °C zur Verbindung der Formel III umsetzt.

7. Verfahren nach Anspruch 1, dadurch gekennzeichnet, dass man die Carbonsäureamid Dimethylformamid verwendet.

8. Verbindungen der Formel III gemäss Anspruch 1.

**Claims**

1. A process for producing a compound of the formula I

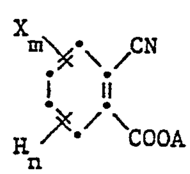

(I)

wherein X is halogen, A is alkyl or phenyl, m is 1 to 4, and n is zero to 3, the sum of m + n being 4, which process comprises reacting the compound of the formula II

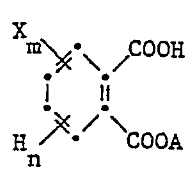

(II)

in which the symbols X, A, m and n are as defined above, in the sole presence of at least a stoichiometric amount of chlorosulfonylisocyanate, to give the compound of the formula III

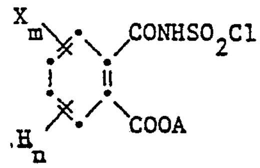

(III)

in which the symbols X, A, m and n are as defined above ; and converting the resultant compound of the formula III by means of a carboxylic acid amide into the compound of the formula I.

2. A process according to claim 1, wherein the starting material used is a compound of the formula II in which X is chlorine, A is $C_1$-$C_4$alkyl or unsubstituted phenyl, m is a number from 2 to 4, and n is a number from zero to 2.

3. A process according to claim 1, wherein the starting material used is a compound of the formula II in which X is chlorine, A is methyl, m is a number from 2 to 4, and n is a number from zero to 2.

4. A process according to claim 1, wherein the starting material used is 3,4,5,6-tetrachloro-2-methoxycarbonylbenzoic acid.

5. A process according to claim 1, wherein the chlorosulfonylisocyanate is used in amounts of 3 to 10 mol equivalents, based on the acid to be reacted.

6. A process according to claim 1, wherein the compound of the formula II is reacted at a temperature in the range from 20° to 60 °C to give the compound of the formula III.

7. A process according to claim 1, wherein the carboxylic acid amide used is dimethylformamide.

8. A compound of the formula III according to claim 1.

**Revendications**

1. Procédé de préparation de composés répondant à la formule I :

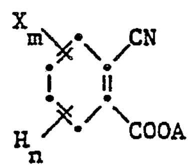

(I)

dans laquelle X représente un halogène, A représente un radical alkyle ou phényle, m représente un nombre de 1 à 4 et n représente un nombre de 0 à 3, la somme (m + n) étant égale à 4, procédé caractérisé en ce qu'on fait réagir un composé répondant à la formule II :

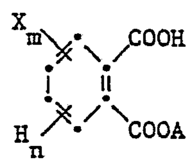

(II)

dans laquelle les symboles X, A, m et n ont les significations précédemment données, en opérant seulement en présence d'une quantité au moins égale à la quantité stœchiométrique d'isocyanate de chlorosulfonyle, de manière à obtenir un composé répondant à la formule III :

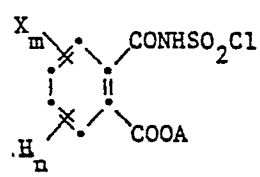

(III)

dans laquelle les symboles X, A, m et n ont les significations qui ont été données ci-dessus, et on transforme le composé de formule III ainsi obtenu, au moyen d'un carboxamide, en un composé de formule I.

2. Procédé selon la revendication 1 caractérisé en ce qu'on utilise, comme corps de départ, un composé de formule II dans lequel X représente le chlore, A représente un radical alkyle contenant de 1 à

4 atomes de carbone ou un radical phényle non substitué, m représente un nombre de 2 à 4 et n représente un nombre de 0 à 2.

3. Procédé selon la revendication 1 caractérisé en ce qu'on utilise, comme corps de départ, un composé de formule II dans lequel X représente le chlore, A un radical méthyle, m un nombre de 2 à 4 et n un nombre de 0 à 2.

4. Procédé selon la revendication 1 caractérisé en ce qu'on utilise, comme corps de départ, l'acide tétrachloro-3,4,5,6 méthoxycarbonyl-2 benzoïque.

5. Procédé selon la revendication 1 caractérisé en ce qu'on utilise l'isocyanate de chlorosulfonyle en une quantité représentant de 3 à 10 équivalents molaires relativement à l'acide à faire réagir.

6. Procédé selon la revendication 1 caractérisé en ce qu'on fait réagir le composé de formule II à une température de 20 à 60 °C de manière à obtenir le composé de formule III.

7. Procédé selon la revendication 1 caractérisé en ce qu'on utilise comme carboxamide le diméthylformamide.

8. Composés de formule III selon la revendication 1.